# EUROPEAN PATENT APPLICATION

(11) **EP 1 541 099 A1**
(43) Date of publication of application: **15.06.2005**
(21) Application number: 04460054.2
(22) Date of filing: 09.12.2004
(51) Int. Cl.: A61F 5/455

(54) **Urinary assistance device**

(30) Priority: 12.12.2003 PL 36303903
(71) Applicant: Szlowieniec, Zofia, 41-200 Sosnowiec (PL); Diakonow, Janusz, 44-200 Rybnik (PL)
(72) Inventor: Szlowieniec, Zofia, 41-200 Sosnowiec (PL); Diakonow, Janusz, 44-200 Rybnik (PL)
(74) Representative: Bogacki, Grzegorz

(57) **Abstract**

The present invention refers to the pad intended to urination in a standing posture, when there are no sufficient hygienic conditions for urination in a sitting posture, e.g. in public toilet or means of transport. Frequently, when toilets are kept in due cleanness and order, there may occur various inhibitions and fears for urination in sitting postures, mainly in women. Such pad has been made of a uniform flexible material and essentially consists of the chamber (1) for urination and connected to this chamber the conical sleeve (2) for removal urine outside.

## Description

The objective of this invention is the pad for hygienic urination, particulary designed by now for application during the hygienic urination by women in a standing posture.

There are numerous different situations, frequently and mostly the necessary ones in which no sufficiently hygienic strictly prescribed conditions for inconvenience of urine in a siting posture have been effectively achieved yet, e.g. during voyages conditions, in route, in health service institutions and/or public Medical Assistance Centers. For instance, the serious problems connected with hygienic urination are also generally encountered during urination in normal standing posture in public toilets during journeys in any means of transport, despite the keeping public facilities clean, in good conditions and/or due cleanness state. However, it is deemed that these inhibitions and impending fear in such cases may be linked not only with sitting posture, but also with a standing posture. Similar problems can be also encountered at the disabled persons with defective body organs of movement, and suffering excessive obesity.

The objective of the present invention is to solve such technical-hygienic problems by creating a very uncomplicated and inexpensive a single usage pad - for applying indirectly and problem less for hygienic urination purposes, to be utilized without any difficulties for the hygienic urination, without a necessity of sitting, but in the standing postures, as exemplified in the above-mentioned situations, effectively to simultaneously provide for maximum hygienic conditions, comfort and conveniences.

The essence of the invention, the pad made of flexible and impermeable material, consists of the chamber for urination and connected to it the conical sleeve for removal of urine outside, which has in the cross-section a shape similar to that a circle or an ellipse, and at one side is provided with outlet ending and at another side ending in the larger sized cross-section, connected to the chamber for urination. The upper edges of the lateral walls of the chamber, forming simultaneously the open inlet aperture for urination, of an arched shape running askew along the longitudinal axis of the pad for hygienic urination, while the height of the back part of the upper edges are always smaller that the height opposite to the front part. The present invention predicts that the front part of the chamber is connected to the outlet ending by means of straight line, forming an extended shape of the sleeve for removal of urine. In both the lateral walls, the discussed pad for hygienic urination has bending concave lines, running along the conical sleeve from the outlet ending to the upper arched form edges of the lateral walls, and of the chamber forming polygonal configuration of the sleeve in the cross-section. Furthermore, the lateral walls of the pad for hygienic urination have a short-sized bending concave lines in the front, upper part of the chamber, while the back part has been equipped with harmonic folded wall.

Such pad for hygienic urination facilitates to remove urine in a standing posture under unfavorable hygienic conditions.

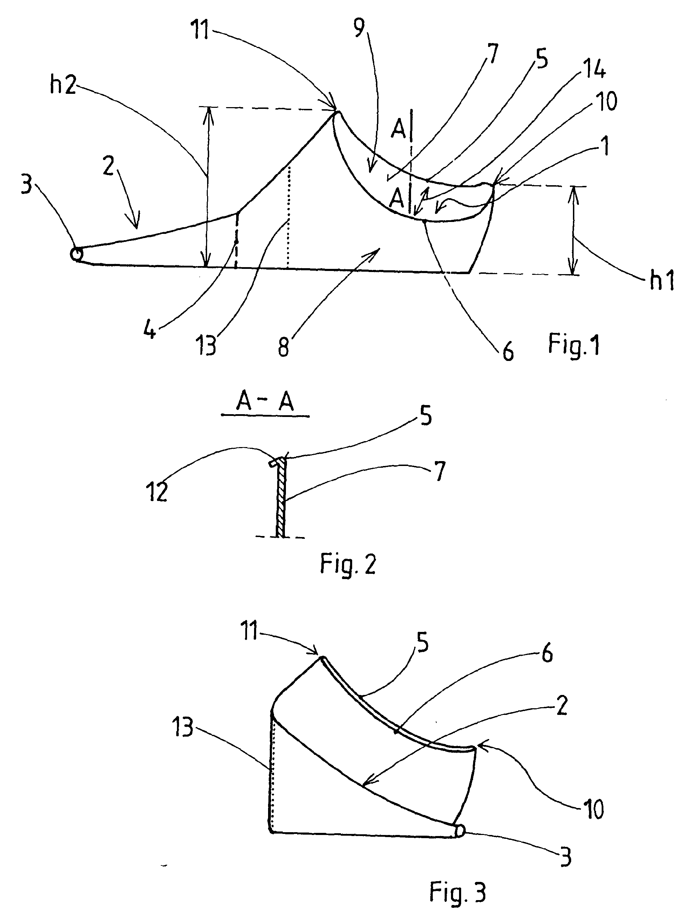

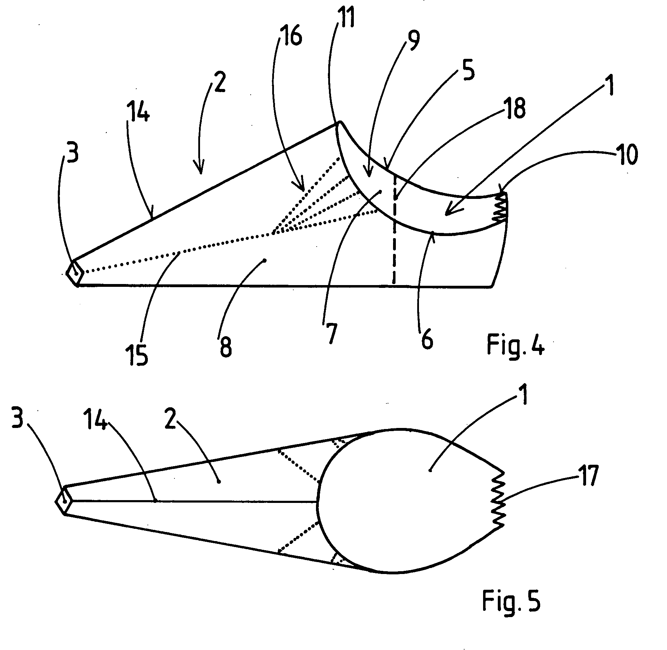

The invention is explained in a more detailed way in accordance with a drawing. **Fig.1** presents as an example of such pad for hygienic urination from the end elevation and-partially from the axonometric projection, while fig. 2 depicts a fragment of the edge of the lateral wall in the vertical section along the section line A-A as marked on **fig.1.** Fig. 3 represents a view upon the folded pad for hygienic urination bent approx. half-way secured lengthways for comfort by carrying it without any problems in women's bags or briefcases. Fig. 4 exemplifies an embodiment of the invention of the pad provided with extended sleeve intended for removal of urine. Fig. 5 presents the pad for hygienic urination in the top view.

According to the present invention, the pad is ad advantageously made through a shape pass of a single sheet of material. The edges can be optionally connected, by fixing by clamps, gluing together, stitching or spot welding, thus shaping the pad after its unfolding, in usable form, depicted in the top view in fig.5 of the drawing.

Such formed pad for hygienic urination consists of a chamber **1** intended for comfortable urination and a conical sleeve **2** linked to the chamber **1**, removing urine outside. The sleeve 2 has in its cross-section the shape similar to circular, elliptical and/or polygonal, and has the ending **3** at one side, and at the another side the ending **4**, marked by dashed line, of significantly larger dimensions in the cross-sections, connected to the chamber **1** intended for urination. The upper edges 5 or 6 of the lateral walls **7** and 8 of the chamber **1**, form simultaneously an open, inlet aperture **9** for urination, which has been invented in the form of arch wise opening askew along the longitudinal axis of the pad for hygienic urination, in such a manner that the height **h1** of the back part **10** of these edges 5 and 6 shall be always smaller than the height **h2** of the front part **11**.

It is preferable that the edges **5** and **6** of the walls of the chamber **1** may contain small-sized bending flanges **12** at the external side or not depicted in figures, small covers made of permeable material to guarantee improved, tight intimate contact up to the human body of the upper perineum during urination.

The present invention also foresees that the front part **11** of the chamber **1** has been connected in the top with the outlet ending 3, by means of the straight line **14** forming an extended shape of the conical sleeve **2** for removal urine. Both lateral walls have the bending concave lines **15**, running along the conical sleeve **2** from the outlet ending **3** up to the upper arch wise edges **5**, **6** of the chamber walls **7**, **8**, forming the polygonal configuration of the specified sleeve in the cross-section. Furthermore, the lateral walls **7**, **8** are provided with bending concave lines 16 with reduced length in the front upper part of the chamber. Such bending concave lines **15**, **16** are represented on the drawing by dash lines enabling to preferably facilitate the configuration of the chamber **1** for the hygienic urination. For this purpose, the invention also foresees that the back part of the chamber **1** has been formed as a harmonic folded wall **17**.

Dashed lines **13** and **18** in fig. 1 and 4 exemplify the places of the pad bending in a single package, as shown on fig. 3, to make it possible keep it in women's bags and/or briefcases. The pad can be made optionally of thin, flexible cardboard or stiffened material impermeable to urine. If required, the package shall be straightened and can deviate the lateral walls **7**, **8** to such a degree that the chamber **1** may be formed, as shown in fig. 5. In such configuration the pad of hygienic urination may be placed under the crotch, lightly pressing the pad up.

## Claims

1. A pad for hygienic urination in a standing posture, made of flexible and impermeable material, is **characterized in that** the pad for hygienic urination consists of the chamber /**1**/ for urination and connected to it the conical sleeve /**2**/ for removal of urine outside, which has in the cross-section a shape similar to that of the circle or ellipse, and at one side is provided with outlet ending /**3**/ and at another side ending /**4**/ in the larger-sized cross-section, connected to the chamber /**1**/ for urination, whereby the upper edges /**5**,**6**/ of the lateral walls /**7,6**/ of the chamber /**1**/, forming simultaneously the open inlet aperture /**9**/ for urination, of an arched shape running askew along the longitudinal axis of the pad for hygienic urination, while the height /**h1**/ of the back part /**10**/ of the upper edges /**5,6**/ are always smaller that the height /**h2**/ opposite to the front part /**11**/.

2. The pad for hygienic urination, according to the claim 1, **characterized in that** the edges /**5,6**/ of the lateral walls /**7,8**/ of the chamber /**1**/ has been provided with bending flanges /**12**/.

3. The pad for hygienic urination, according to the claim 1, is **characterized in that** the edges /**5,6**/ of the lateral walls /**7,8**/ of the chamber /**1**/ have covers made of permeable material.

4. The pad for hygienic urination, according to the claim 1, **characterized in that** the front part /**11**/ of the chamber /**1**/ is linked upwards to the outlet ending /**3**/ by means of straight line /14/, forming an extended shape of the conical sleeve /**2**/ for removal of urine.

5. The pad for hygienic urination according to the claim 1, **characterized in that** the both lateral walls it has the bending concave lines /**15**/, running along the conical sleeve /**2**/ from the outlet ending /**3**/ up to the upper arched form edges /**5,6**/ of the lateral walls /**7** and **8**/ of the chamber, forming the polygonal configuration of the sleeve in the cross-section.

6. The pad for hygienic urination, according to the claim 1, **characterized in that** the lateral walls /**7,8**/ have the bending concave lines /**16**/ in the front, upper part of the chamber /**1**/.

7. The pad for hygienic urination, according to the claim 1, **characterized in that** the back part of the chamber /**1**/ has been provided with harmonic folded wall /17/.
